Europäisches Patentamt

European Patent Office

Office européen des brevets

(19)

(11) Publication number: **0 133 591**
**B1**

(12) **EUROPEAN PATENT SPECIFICATION**

(45) Date of publication of patent specification: **13.07.88**

(21) Application number: **84201121.5**

(22) Date of filing: **31.07.84**

(51) Int. Cl.⁴: **C 07 C 2/84,** C 07 C 15/02,
B 01 J 23/08, B 01 J 37/16,
C 07 C 2/74

(54) **Process for the preparation of an aromatic hydrocarbon mixture.**

(30) Priority: **08.08.83 NL 8302788**

(43) Date of publication of application:
**27.02.85 Bulletin 85/09**

(45) Publication of the grant of the patent:
**13.07.88 Bulletin 88/28**

(84) Designated Contracting States:
**BE DE FR GB IT NL**

(56) References cited:
**EP-A-0 043 695**
**EP-A-0 107 875**
**EP-A-0 107 876**
**EP-A-0 107 877**
**GB-A-2 117 367**
**US-A-4 180 689**

(73) Proprietor: **SHELL INTERNATIONALE
RESEARCH MAATSCHAPPIJ B.V.
Carel van Bylandtlaan 30
NL-2596 HR Den Haag (NL)**

(72) Inventor: **Kieffer, Eduard Philip
Badhuisweg 3
NL-1031 CM Amsterdam (NL)**

(74) Representative: **Aalbers, Onno et al
P.O. Box 302
NL-2501 CH The Hague (NL)**

Courier Press, Leamington Spa, England.

## Description

The invention relates to a process for the preparation of an aromatic hydrocarbon mixture by contacting one or more paraffins with two, three or four carbon atoms per molecule, or aliphatic hydrocarbon mixtures containing more than 50 %w of said paraffins with a catalyst comprising gallium supported on a carrier and a crystalline metal silicate which

a) has been prepared by crystallization from an aqueous mixture which, in addition to the components needed for the synthesis of the silicate, comprises one or more compounds of a trivalent metal Y chosen from the group formed by aluminium, iron, cobalt and chromium and, if desired, one or more gallium compounds, in such quantities that in the formula which represents the composition of the silicate expressed in moles of the oxides, the $SiO_2/(Y_2O_3+Ga_2O_3)$ molar ratio is 10—500 and the $Ga_2O_3/Y_2O_3$ molar ratio is lower than 1, and

b) after one hour's calcination in air at 500°C has an X-ray powder diffraction pattern in which the strongest lines are the four lines mentioned in Table A

### TABLE A

$d(Å)$
11.1 ±0.2
10.0 ±0.2
3.84±0.07
3.72±0.06

in which catalyst the quantity of gallium supported on a carrier amounts to 1—10 %w, calculated on the quantity of crystalline metal silicate present in the catalyst, and which catalyst has been subjected once or several times to a two-step treatment comprising a first step in which the catalyst is treated at an elevated temperature, followed by a second step in which the catalyst is contacted for at least 15 minutes and at a temperature of 350—700°C with an oxidizing gas which contains at least 5 %w oxygen.

Such a process is known from EP—A—107,877 in the name of the present Applicant and forming a state of the art according to Article 54(3) EPC, in which process coke is deposited on the catalyst in the first step by contacting the catalyst at a temperature of 350—700°C with a hydrocarbon (mixture) followed by a coke-burn off step with an oxygen-containing gas.

Furthermore it is known from EP—A—43,695 to restore the activity of a catalyst composition comprising an alumunosilicate having a gallium compound deposited thereon and/or having been subjected to ion exchange with gallium ions, by heating the catalyst in a first step in an oxidizing atmosphere and subsequently in a second step in a reducing atmosphere.

A major problem, however, occurring when applying the particular gallium-containing supported catalysts as described hereinbefore in a process for the preparation of aromatic hydrocarbons and, in addition, hydrogen, is the relatively low $C_5^+$ selectivity, $H_2$ selectivity and conversion generally attained with such catalysts.

Surprisingly, it has now been found that the aforementioned problem can be overcome by subjecting said catalysts to a specific two-step treatment, of which the first step is carried out with a hydrogencontaining reducing gas, provided that the gallium-content of the catalysts meets an additional criterion.

The invention therefore relates to a process as described in the preamble which is characterized in that a catalyst containing 0.3—10 %w of gallium supported on a carrier, calculated on the sum of the quantity of crystalline metal silicate present in the catalyst and the quantity of other material, if any, used as carrier for the gallium, is contacted in the first step for at least 15 minutes at a temperature of 400—650°C with a reducing gas containing at least 20 %v hydrogen.

In addition, the present Applicant has filed on even date EP—A—134058 relating to a process for the preparation of an aromatic hydrocarbon mixture with the use of a crystalline gallium silicate catalyst which may comprise, if desired, a similar trivalent metal Y as the catalysts employed in the process according to the present invention. However, the $Y_2O_3/Ga_2O_3$ molar ratio of the respective compounds, expressed in moles of the oxides, present in the starting mixture from which said crystalline gallium silicates are prepared, is lower than 1, whereas in the starting mixture used for the preparation of the crystalline metal silicates applied in the process according to the present invention, the $Ga_2O_3/Y_2O_3$ molar ratio is lower than 1.

The liquid hydrocarbon mixtures obtained in the present conversion boil substantially in the gasoline range and have a very high octane number. They are therefore excellenty suitable for use as motor gasoline or as mixing components for motor gasolines.

If the starting material is an aliphatic hydrocarbon mixture which, in addition to the paraffins mentioned, contains other aliphatic hydrocarbons as well, this mixture may contain, inter alia, methane, ethene, propene, butene, isobutene, butadiene and paraffins and olefins with five or more carbon atoms per molecule. In the process according to the invention the starting material preferred is a feed which consists more than 75 %w, and in particular substantially completely, of one or more paraffins having three

2

or four carbon atoms per molecule. A feedstock which is very suitable for use in the process is a mixture of paraffins with three and four carbon atoms per molecule obtained as a by-product in the production of mineral oil.

The process according to the invention is preferably carried out at a temperature of 350—700°C and in particular of 450—650°C, a pressure of 1—20 bar and in particular of 1—10 bar and a space velocity of 0.1—10 kg.kg$^{-1}$.hour$^{-1}$ and in particular of 0.5—5 kg.kg$^{-1}$.hour$^{-1}$.

In the process according to the invention the feed is contacted with a catalyst containing a crystalline metal silicate which is defined, among other things, by the X-ray powder diffraction pattern which the silicate shows after one hour's calcination in air at 500°C. In this pattern the strongest lines should be the four lines mentioned in Table A. The complete X-ray powder diffraction pattern of a typical example of the present crystalline metal silicates after one hour's calcination in air at 500°C is given in Table B.

TABLE B

| d(Å) | Rel. int. | d(Å) | Rel. int. |
|---|---|---|---|
| 11.1 | 100 | 3.84 (D) | 57 |
| 10.0 (D) | 70 | 3.72 (D) | 31 |
| 8.93 | 1 | 3.63 | 16 |
| 7.99 | 1 | 3.47 | <1 |
| 7.42 | 2 | 3.43 | 5 |
| 6.68 | 7 | 3.34 | 2 |
| 6.35 | 11 | 3.30 | 5 |
| 5.97 | 17 | 3.25 | 1 |
| 5.70 | 7 | 3.05 | 8 |
| 5.56 | 10 | 2.98 | 11 |
| 5.35 | 2 | 2.96 | 3 |
| 4.98 (D) | 6 | 2.86 | 2 |
| 4.60 | 4 | 2.73 | 2 |
| 4.35 | 5 | 2.60 | 2 |
| 4.25 | 7 | 2.48 | 3 |
| 4.07 | 2 | 2.40 | 2 |
| 4.00 | 4 | | |

(D)=doublet

The catalysts used in the process according to the invention are catalysts comprising the prescribed percentage of gallium supported on a carrier and in addition a crystalline metal silicate of a special structure which has a $SiO_2/(Y_2O_3+Ga_2O_3)$ molar ratio of 10—500. A carrier for the gallium that may suitably be used is the crystalline metal silicate. The gallium is preferably deposited on the crystalline metal silicate by impregnation or ion exchange starting from an aqueous solution of a gallium compound, such as gallium nitrate. Very suitable catalysts for carrying out the present conversion may also be prepared by depositing the gallium on a conventional carrier, such as silica and mixing the gallium-loaded carrier with the crsytalline metal silciate. Mixtures of gallium supported on silica as the carrier and gallium supported on the crystalline metal silicate as the carrier are also suitable for use as catalyst in the present conversion. In the process according to the invention preference is given to catalysts in which the carrier for the gallium is exclusively the crystalline metal silicate and in which the gallium has been deposited on the crystalline metal silicate by impregnation or ion exchange, as well as to catalysts in which the carrier for the gallium is

3

exclusively an amorphous material, which gallium-loaded carrier is present in the catalyst in admixture with the crystalline metal silicate.

The crystalline metal silicate which is present in the catalysts as the carrier for the gallium and/or as a mixing component for a gallium-loaded carrier has a $SiO_2/(Y_2O_3+Ga_2O_3)$ molar ratio of 10—500. To be quite on the safe side it is pointed out that in determining the $SiO_2/(Y_2O_3+Ga_2O_3)$ molar ratio only the quantity of gallium should be taken into account which may have been incorporated in the silicate during the synthesis of the silicate by crystallization from an aqueous mixture containing one or more gallium compounds. Neither gallium which has been deposited on the ready crystalline metal silicate after the synthesis, for instance by impregnation or ion exchange, nor gallium which is present in the catalyst supported on an amorphous carrier plays a role in the determination of the $SiO_2/(Y_2O_3+Ga_2O_3)$ molar ratio of the crystalline metal silicate.

In the process according to the invention preference is given to the use of a catalyst comprising a crystalline metal silicate with a $SiO_2/(Y_2O_3+Ga_2O_3)$ molar ratio of 25—250.

As regards the number of times that the two-step treatment should be carried out in order to obtain a catalyst with opimum performance in the present conversion, the following may be remarked.

In general, the performance of the present catalysts can be raised to an optimum level by subjecting them at most three times to the two-step treatment. The investigation has shown that in contrast with related gallium-containing catalysts where there was seen to be a relation between the gallium content of the catalysts and the number of times that the two-step treatment had to be carried out in order to achieve optimum performance, no such relation exists where the present catalysts are concerned. The investigation has further shown that, also in contrast with the afore-mentioned related gallium-containing catalysts, for which a considerable reduction of the number of times that the two-step treatment has to be carried out can be achieved by carrying out preceding calcination at a temperature between 600 and 1000°C, in the case of the present catalysts, such a pre-treatment is detrimental to the catalyst performance.

In the first step of the two-step treatment the catalysts should be contacted for at least 15 minutes and at a temperature of 400—650°C with a reducing gas containing at least 20 %v hydrogen. The first step is preferably carried out at a temperature of 475—575°C and using a reducing gas containing at lesat 40 %v hydrogen. The first step can very suitably be carried out by using a gas which, in addition to hydrogen, contains either substantially nitrogen, or substantially carbon monoxide, or substantially $C_4^-$ hydrocarbons. Suitable gases which in addition to hydrogen contain substantially carbon monoxide may be obtained as a synthesis gas, from a heavy carbonaceous material, such as coal, by gasification, or from light hydrocarbons, such as natural gas, by steam reforming or partial oxidation. Suitable gases which in addition to hydrogen contain substantially $C_4^-$ hydrocarbons may be obtained as a byproduct in the catalytic conversion of hydrocarbons in the presence of hydrogen, such as cracking, isomerisation and reforming.

In the second step of the two-step treatment the catalyst should be contacted for at least 15 minutes and at a temperature of 350—700°C with an oxidizing gas containing at least 5 %v oxygen. The second step is preferably carried out at a temperature of 475—575°C and by using an oxidizing gas containing at least 10 %v oxygen. The second step can very suitably be carried out by using a gas which in addition to oxygen, contains either substantially nitrogen, or substantially nitrogen, carbon monoxide and carbon dioxide. A suitable gas which in addition to oxygen contains substantially nitrogen, is air. Suitable gases which in addition to oxygen contain substantially nitrogen, carbon monoxide and carbon dioxide are exhaust gases produced in the removal by excess air of coke from deactivated hydrocarbon conversion catalysts. The first and the second step of the two-step treatment are preferably carried out at the same temperature.

The preparation of the crystalline metal silicates used in the process according to the invention may very suitably be carried out starting from an aqueous mixture comprising the following compounds: one or more compounds of an alkali metal (M), one or more organic nitrogen compounds (RN) which contain an organic cation or from which an organic cation is formed during the preparation of the silicate, one or more silicon compounds, one or more compounds of a trivalent metal Y and, if desired one or more gallium compounds. The preparation is carried out by maintaining the mixture at an elevated temperature under the silicate has formed and subsequently separating the silicate crystals from the mother liquor and washing, drying and calcining the crystals. In the aqueous mixture from which the silicates are prepared the various compounds should be present in the following molar ratios expressed—with the exception of the organic nitrogen compounds—in moles of the oxides:

$$M_2O \quad : SiO_2 = 0.01—0.35,$$
$$RN \quad : SiO_2 = 0.02—1.0,$$
$$SiO_2 \quad : (Y_2O_3+Ga_2O_3) = 10—750,$$
$$Ga_2O_3 \quad : Y_2O_3 < 1, \text{ and}$$
$$H_2O \quad : SiO_2 = 5—65.$$

In the preparation of the silicates the base mixture may very suitably be a mixture containing a quaternary ammonium compound as organic nitrogen compound, a sodium compound as alkali metal compound and amorphous silica as silicon compound.

In the process according to the invention preference is given to the use of crystalline metal silicates

**0 133 591**

which have been prepared by crystallization from an aqueous mixture which contains no gallium compounds and which contains at least one or more aluminium compounds as compounds of a trivalent metal Y.

The silicates prepared as described hereinbefore contain alkali metal ions. By using suitable exchange methods these may be replaced by other cations, such as hydrogen ions or ammonium ions. The crystalline metal silicates used in the process according to the invention preferably have an alkali metal content of less than 0.05 %w. In the process according to the invention the compositions comprising gallium supported on a carrier as well as a crystalline metal silicate of a special structure may be used per se or combined with a binder material, such as kaolin or bentonite.

The invention is now elucidated with the aid of the following example.

Example

Two crystalline aluminium silicates (Silicates 1 and 2) were prepared by heating mixtures of NaOH, amorphous silica, $(C_3H_7)_4NOH$ and $NaAlO_2$ in water, in an autoclave under autogeneous pressure, at 150°C for 24 hours. After cooling of the reaction mixtures the silicates formed were filtered off, washed with water until the pH of the wash water was about 8 and dried at 120°C. After one hour's calcination in air at 500°C Silicates 1 and 2 had the following properties

a) an X-ray powder diffraction pattern substantially corresponding with that mentioned in Table B, and
b) a $SiO_2/Al_2O_3$ molar ratio of 225 for Silicate 1 and 175 for Silicate 2.

From Silicates 1 and 2 were prepared Silicates I and II respectively, by boiling Silicates 1 and 2 with a 1.0 molar $NH_4NO_3$ solution, washing with water, boiling again with a 1.0 molar $NH_4NO_3$ solution and washing, drying at 120°C and calcination at 500°C.

Catalysts I and II were prepared from Silicates I and II as follows:

Catalyst I

This catalyst was prepared by impregnating Silicate I with an aqueous solution of gallium nitrate. Catalyst I contained 2 %w gallium.

Catalyst II

This catalyst was prepared by mixing Silicate II in a weight ratio of 1:1 with a composition which contains 3 %w gallium on silica and had been obtained by impregnation of silica with an aqueous solution of gallium nitrate. Catalyst II contained 1.5 %w gallium.

Samples of Catalysts I and II were subjected several times to a two-step treatment comprising a step in which the catalyst was contacted for 30 minutes at a temperature of 550°C and a pressure of 1.5 bar with a $H_2/N_2$ mixture in a 1:1 volume ratio, followed by a second step in which the catalyst was contacted for 1 hour at a temperature of 550°C and a pressure of 1.5 bar with air. From Catalysts I and II were thus produced Catalysts IA, IB and IIA, respectively.

Catalysts I—IB, II and IIA were tested in five experiments (Experiments 1—5) in the preparation of $C_5^+$ aromatic hydrocarbon mixtures starting from n-butane. The experiments were carried out in a reactor containing a fixed catalyst bed. Experiments 1—3 were carried out at a temperature of 550°C, a pressure of 5 bar and a space velocity of 8 kg.kg$^{-1}$.hour$^{-1}$ and Experiments 4 and 5 at a temperature of 575°C, a pressure of 1.5 bar and a space velocity of 2 kg.kg$^{-1}$.hour$^{-1}$. The results of the experiments are listed in Table C. Table C also indicates how many times each of the catalysts has been subjected to the two-step treatment.

Of the experiments mentioned in Table C Experiments 2, 3 and 5 are experiments according to the invention. These experiments were carried out using catalysts comprising gallium supported on a carrier as well as a crystalline metal silicate of a special structure, which catalysts had been subjected to at most four two-step treatments according to the invention. These catalysts show high activity and $H_2$ selectivity as well as acceptable $C_5^+$ selectivity. Comparison of the results of Experiments 1—3 shows that the catalyst performance is much enhanced if the catalyst is subjected to the two-step treatment twice, but that the performance shows no marked further improvement when the two-step treatment is repeated four times.

Experiments 1 and 4 fall outside the scope of the invention. They have been included in the patent application for comparison. Experiments 1 and 4 were carried out using catalysts comprising gallium supported on a carrier and a crystalline metal silicate of a special structure, but these catalysts had not been subjected to a two-step treatment according to the invention.

**0 133 591**

TABLE C

| Experiment No. | 1 | 2 | 3 | 4 | 5 |
|---|---|---|---|---|---|
| Catalyst No. | I | IA | IB | II | IIA |
| Number of times that catalyst was subjected to two-step treatment | — | 2 | 4 | — | 3 |
| Conversion, %w | 40 | 54 | 57 | 70 | 68 |
| Product selectivity, %w on converted material | | | | | |
| $H_2$ | 3.8 | 4.5 | 5.2 | 3.0 | 5.0 |
| $C_1—C_3$ | 70.2 | 58.5 | 53.3 | 74.8 | 50.7 |
| $iso\text{-}C_4^0$ | 4.0 | 3.1 | 5.5 | 4.2 | 4.6 |
| $\Sigma C_4^=$ | 6.8 | 4.2 | 4.4 | 5.2 | 7.2 |
| $C_5^+$ | 15.2 | 29.7 | 31.6 | 12.8 | 32.5 |

**Claims**

1. A process for the preparation of an aromatic hydrocarbon mixture by contacting one or more paraffins with two, three or four carbon atoms per molecule, or aliphatic hydrocarbon mixtures containing more than 50 %w of said paraffins with a catalyst comprising gallium supported on a carrier and a crystalline metal silicate which

a) has been prepared by crystallization from an aqueous mixture which, in addition to the components needed for the synthesis of the silicate, comprises one or more compounds of a trivalent metal Y chosen from the group formed by aluminium, iron, cobalt and chromium and, if desired, one or more gallium compounds, in such quantities that in the formula which represents the composition of the silicate expressed in moles of the oxides, the $SiO_2/(Y_2O_3+Ga_2O_3)$ molar ratio is 10—500 and the $Ga_2O_3/Y_2O_3$ molar ratio is lower than 1, and

b) after one hour's calcination in air at 500°C has an X-ray powder diffraction pattern in which the strongest lines are the four lines mentioned in Table A

TABLE A

d(Å)
11.1 ±0.2
10.0 ±0.2
3.84±0.07
3.72±0.06

in which catalyst the quantity of gallium supported on a carrier amounts to 1—10 %w, calculated on the quantity of crystalline metal silicate present in the catalyst, and which catalyst has been subjected once or several times to a two-step treatment comprising a first step in which the catalyst is treated at an elevated temperature, followed by a second step in which the catalyst is contacted for at least 15 minutes and at a temperature of 350—700°C with an oxidizing gas which contains at least 5 %v oxygen, characterized in that a catalyst containing 0.3—10 %w of gallium supported on a carrier, calculated on the sum of the quantity of crystalline metal silicate present in the catalyst and the quantity of other material, if any, used as carrier for the gallium, is contacted in the first step for at least 15 minutes at a temperature of 400—650°C with a reducing gas containing at least 20 %v hydrogen.

2. A process as claimed in claim 1, characterized in that the reducing gas contains at least 40 %v hydrogen.

3. A process as claimed in claim 1 or 2, characterized in that the two steps of the two-step treatment are carried out at a temperature of 475—575°C.

4. A process as claimed in any one of claims 1—3, characterized in that the two steps of the two-step treatment are carried out at the same temperature.

6

# 0 133 591

**Patentansprüche**

1. Ein Verfahren zur Herstellung einer aromatischen Kohlenwasserstoffmischung durch Kontaktieren eines oder mehrerer Paraffine mit zwei, drei oder vier Kohlenstoffatomen im Molekül oder von aliphatischen Kohlenwasserstoffmischungen, enthaltend mehr als 50 Gewichtsprozent der genannten Paraffine, mit einem Katalysator enthaltend Gallium auf einer Trägersubstanz und ein kristallines Metallsilikat, welches

a) hergestellt wurde durch Kristallisation aus einer wäßrigen Mischung, welche zusätzlich zu den für die Synthese des Silikats notwendigen Bestandteilen eine oder mehrere Verbindungen eines dreiwertigen Metalls Y, ausgewählt aus der Gruppe bestehend aus Aluminium, Eisen, Kobalt und Chrom und, falls gewünscht, eine oder mehrere Galliumverbindungen, in solchen Mengen enthält, daß in der Formel, welche die Zusammensetzung des Silikats ausgedrückt in Mol der Oxide darstellt, das molare Verhältnis $SiO_2$ : $(Y_2O_3+Ga_2O_3)$ 10 bis 500 beträgt und das molare Verhältnis $Ga_2O_3/Y_2O_3$ weniger als 1 betragt, und

b) nach einstündigem Kalzinieren an Luft bei 500°C ein Röntgenpulverstreudiagramm aufweist, in welchem die vier in Tabelle A genannten Linien am deutlichsten ausgeprägt sind

### TABELLE A

$d(Å)$
11,1 ±0,2
10,0 ±0,2
3,84±0,07
3,72±0,06

bei welchem Katalysator die Menge an Gallium auf einem Träger von 1 bis 10 Gewichtsprozent beträgt, bezogen auf die Menge an kristallinem Metallsilikat, das im Katalysator vorliegt, und welcher Katalysator einmal oder mehrere Male einer Zweistufen-Behandlung unterworfen wurde, welche eine erste Stufe umfaßt, in welcher der Katalysator bei erhöhter Temperatur behandelt wird, gefolgt von einer zweiten Stufe, in welcher der Katalysator mindestens 15 Minuten und bei einer Temperatur von 350 bis 700°C mit einem oxidierenden Gas, welches mindestens 5 Volumenprozent Sauerstoff enthält, kontaktiert wird, dadurch gekennzeichnet, daß ein Katalysator enthaltend 0,3 bis 10 Gewichtsprozent Gallium auf einer Trägersubstanz, bezogen auf die Summe der Menge des im Katalysator vorliegenden kristallinen Metallsilikats und der Menge an anderem Material, falls vorhanden, das als Träger für das Gallium eingesetzt wurde, in der ersten Stufe mindestens 15 Minuten lang bei einer Temperatur von 400 bis 650°C mit einem reduzierenden Gas, enthaltend mindestens 20 Volumenprozent Wasserstoff, kontaktiert wird.

2. Ein Verfahren wie in Anspruch 1 beansprucht, dadurch gekennzeichnet, daß das reduzierende Gas mindestens 40 Volumenprozent Wasserstoff enthält.

3. Ein Verfahren wie in Anspruch 1 oder 2 beansprucht, dadurch gekennzeichnet, daß die zwei Stufen der Zweistufen-Behandlung bei einer Temperatur von 475 bis 575°C durchgeführt werden.

4. Ein Verfahren wie in einem der Ansprüche 1 bis 3 beansprucht, dadurch gekennzeichnet, daß die beiden Stufen der Zweistufen-Behandlung bei gleicher Temperatur durchgeführt werden.

**Revendications**

1. Un procédé de préparation d'un mélange d'hydrocarbures aromatiques par mise en contact d'une ou plusieurs paraffines ayant deux, trois ou quatre atomes de carbone par molécule, ou de mélanges d'hydrocarbures aliphatiques contenant plus de 50 % en poids de ces paraffines avec un catalyseur comprenant du gallium déposé sur un support et un silicate métallique cristalline qui

a) a été préparé par cristallisation à partir d'un mélange aqueux qui, en plus des constituants nécessaires pour la synthèse du silicate, comprend un ou plusieurs composés d'un métal trivalent Y choisi dans le groupe formé par l'aluminium, le fer, le cobalt et le chrome et, si on le désiré, un ou plusieurs composés du gallium, en quantités telles que dans la formule qui représente la composition du silicate exprimée en moles des oxydes, le rapport molaire $SiO_2/(Y_2O_3+Ga_2O_3)$ soit de 10—500 et le rapport molaire $Ga_2O_3/Y_2O_3$ soit inférieur à 1, et

b) après calcination pendant une heure dans l'air à 500°C a un diagramme de diffraction des rayons X par la méthode des poudres dans lequel les lignes les plus intenses sont les quatre lignes mentionnées dans le tableau A

### TABLEAU A

$d(0,1\ nm)$
11,1 ±0,2
10,0 ±0,2
3,84±0,07
3,72±0,06

7

**0 133 591**

la quantité de gallium déposé sur un support dans le catalyseur se montant à 1—10% en poids, calculée par rapport à la quantité de silicate métallique cristallin présente dans le catalyseur, et le catalyseur ayant été soumis une ou plusieurs fois à un traitement en deux étapes comportant une première ètape dans laquelle le catalyseur est traité à une température élevée, suivie d'une seconde étape dans laquelle le catalyseur est mis en contact pendant au moins 15 minutes et à une température de 350—700°C avec un gaz oxydant qui contient au moins 5 % en volume d'oxygène, caractérisé en ce qu'un catalyseur contenant 0,3—10 % en poids de gallium déposé sur un support, en calculant par rapport à la somme de la quantité de silicate métallique cristallin présente dans le catalyseur et de la quantité de toute autre matière éventuellement utilisée comme support pour le gallium, est mis en contact dans la première étape pendant au moins 15 minutes à une température de 400—650°C avec un gaz réducteur contenant au moins 20 % en volume d'hydrogène.

2. Un procédé selon la revendication 1, caractérisé en ce que le gaz réducteur contient au moins 40 % en volume d'hydrogène.

3. Un procédé selon la revendication 1 ou 2, caractérisé en ce que les deux étapes du traitement en deux étapes sont conduites à une température de 475—575°C.

4. Un procédé selon l'une quelconque des revendications 1—3, caractérisé en ce que les deux étapes du traitement en deux étapes sont conduites à la même température.